Europäisches Patentamt
European Patent Office
Office européen des brevets

(11) Veröffentlichungsnummer: **0 299 360 A2**

# EUROPÄISCHE PATENTANMELDUNG

(21) Anmeldenummer: 88110860.9

(22) Anmeldetag: 07.07.88

(51) Int. Cl.4 C07C 43/11 , C07C 43/178 , C07C 41/03 , C07C 43/13

(30) Priorität: 15.07.87 DE 3723323

(43) Veröffentlichungstag der Anmeldung: 18.01.89 Patentblatt 89/03

(84) Benannte Vertragsstaaten: AT BE CH DE ES FR GB GR IT LI NL SE

(71) Anmelder: Henkel Kommanditgesellschaft auf Aktien
Postfach 1100 Henkelstrasse 67
D-4000 Düsseldorf-Holthausen(DE)

(72) Erfinder: Schenker, Gilbert, Dr.
Hermann-Hesse-Strasse 5
D-4006 Erkrath(DE)
Erfinder: Piorr, Robert, Dr.
Kieselei 12
D-4030 Ratingen-Hösel(DE)
Erfinder: Lüttge, Sabine
Marktfelderstrasse 30
D-4050 Mönchengladbach 1(DE)

## (54) Hydroxy-Mischether, Verfahren zu deren Herstellung sowie deren Verwendung.

(57) Die Erfindung betrifft Hydroxyalkylpolyethylen- und Hydroxyalkylpolypropylenglykolether der allgemeinen Formel (I)

$$R^1\text{-}\overset{OH}{CH}\text{-}CH_2\text{-}(O\overset{R^3}{CH}\text{-}CH_2)_n\text{-}OR^2 \qquad (I)$$

in der $R^1$ für einen linearen Alkylrest mit 6 bis 16 C-Atomen, $R^2$ für einen geradkettigen oder verzweigten, gesättigten oder ungesättigten Alkylrest mit 1 bis 22 C-Atomen, $R^3$ für Wasserstoff oder eine Methylgruppe und n für eine Zahl von 0 bis 30 stehen, sowie Mischungen mehrerer derartiger Verbindungen, ein Verfahren zur Herstellung derartiger Hydroxy-Mischether, das dadurch gekennzeichnet ist, daß man geeignete Epoxide mit geeigneten Alkoholalkoxylaten bei erhöhter Temperatur unter Schutzgas in Gegenwart eines Katalysators umsetzt, sowie die Verwendung der genannten Verbindungen oder ihrer Mischungen als schaumdrückende Zusätze in schaumarmen Reinigungsmitteln.

EP 0 299 360 A2

## Hydroxy-Mischether, Verfahren zu deren Herstellung sowie deren Verwendung

Die Erfindung betrifft Hydroxyalkylpolyethylen- bzw. Hydroxyalkylpolypropylenglykolether. ein Verfahren zur Herstellung derartiger Verbindungen sowie deren Verwendung als schaumdrückende Zusätze in schaumarmen Reinigungsmitteln.

Für die Verwendung in Gewerbe und Industrie bestimmte wäßrige Reinigungsmittel. insbesondere solche für die Reinigung von Metall-. Glas-. Keramik- und Kunststoffoberflächen, enthalten in der Regel Substanzen. die unter den vorgegebenen Arbeitsbedingungen Anlaß zu unerwünschter Schaumbildung geben. Solche Substanzen sind beispielsweise Aniontenside oder bei Arbeitstemperatur schäumende nichtionische Tenside. Um der unerwünschten Schaumbildung vorzubeugen. ist es deswegen in nahezu allen Fällen erforderlich. derartigen wäßrigen Reinigungsmitteln Substanzen zuzusetzen. die die unerwünschte Schaumbildung verhindern oder die Tendenz dazu verringern können. Der Einsatz von schaumdrückenden Zusätzen ist in den meisten Fällen auch deswegen erforderlich. weil die von den Substraten abgelösten und sich in den Reinigungsbädern ansammelnden Verunreinigungen zusätzlichen Schaum bilden.

Als schaumdrückende Zusätze werden seit langem Anlagerungsprodukte von Alkylenoxiden an organische Verbindungen, die - vorzugsweise mehrere - reaktive Wasserstoffatome im Molekül besitzen. mit gutem Erfolg eingesetzt. Hier haben sich insbesondere Anlagerungsprodukte von Propylenoxid an aliphatische Polyalkohole (DE-PSen 12 80 455 und 16 21 592). Anlagerungsprodukte von Propylenoxid an aliphatische Polyamine (DE-PSen 12 89 579 und 16 21 593) sowie Anlagerungsprodukte von Ethylenoxid und Propylenoxid an aliphatische Polyamine. insbesondere Ethylendiamin (DE-PS 19 44 569). in der Praxis bewährt. Diese Alkylenoxid-Anlagerungsprodukte besitzen neben einer guten schaumdrückenden Wirkung auch die für die Anwendung in gewerblichen und industriellen Reinigungsmitteln zumeist erforderliche Alkalistabilität. Die Verbindungen dieser Klasse sind jedoch nicht hinreichend biologisch abbaubar und genügen damit nicht den geltenden gesetzlichen Vorschriften (RVO zum Waschmittelgesetz).

Der vorliegenden Erfindung lag die Aufgabe zugrunde. schaumdrückende Substanzen aufzufinden. die in einfacher Weise herstellbar sind und hinsichtlich ihrer anwendungstechnischen Eigenschaften den Substanzen des Standes der Technik zumindest gleichkommen. wenn nicht sie sogar verbessern. Zudem sollten Substanzen zur Verfügung gestellt werden, die bei geringem Anfangsschaum ein gutes Durchhaltevermögen zeigen. also die Schaumbildung bei steigenden Testschäumer-Konzentrationen wirkungsvoll inhibieren. Dabei war der Tatsache Rechnung zu tragen. daß die gesetzlichen Abwasservorschriften eine weitgehende biologische Abbaubarkeit derartiger Substanzen vorschreiben. damit nach Einleitung in die Kanalisation eine Belastung der Abwässer nicht zu befürchten ist.

Die Lösung dieser Aufgaben ging von der Vorstellung aus. neue Produkte zur Verfügung zu stellen. die eine geringe Anfangsschaumhöhe, andererseits jedoch ein gutes Durchhaltevermögen aufweisen.

Die Erfindung betrifft Hydroxyalkylpolyethylen- und Hydroxyalkylpolypropylenglykolether der allgemeinen Formel (I)

$$R^1-\underset{|}{\overset{OH}{C}}H-CH_2-(O\underset{|}{\overset{R^3}{C}}H-CH_2)_n-OR^2 \qquad (I)$$

in der

$R^1$ für einen linearen Alkylrest mit 6 bis 16 C-Atomen.

$R^2$ für einen geradkettigen oder verzweigten. gesättigten oder ungesättigten Alkylrest mit 1 bis 22 C-Atomen.

$R^3$ für Wasserstoff oder eine Methylgruppe und

n für eine Zahl von 0 bis 30

stehen. sowie Mischungen mehrerer derartiger Verbindungen.

Die Erfindung betrifft außerdem ein Verfahren zur Herstellung von Hydroxyalkylpolyethylen- und Hydroxyalkylpolypropylenglykolethern der allgemeinen Formel (I)

$$\overset{OH}{\overset{|}{R^1-CH}}-CH_2-(\overset{R^3}{\overset{|}{OCH}}-CH_2)_n-OR^2 \qquad (I)$$

in der

$R^1$ für einen linearen Alkylrest mit 6 bis 16 C-Atomen,

$R^2$ für einen geradkettigen oder verzweigten, gesättigten oder ungesättigten Alkylrest mit 1 bis 22 C-Atomen,

$R^3$ für Wasserstoff oder eine Methylgruppe und

n für eine Zahl von 0 bis 30

stehen, sowie von Mischungen mehrerer derartiger Verbindungen, das dadurch gekennzeichnet ist, daß man Epoxide der allgemeinen Formel (II)

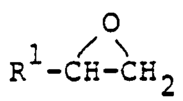

(II)

in der $R^1$ die oben angegebenen Bedeutungen hat, mit Alkoholalkoxylaten der allgemeinen Formel (III)

$$H-(\overset{R^3}{\overset{|}{OCH}}-CH_2)_n-OR^2 \qquad (III)$$

in der $R^2$ und $R^3$ sowie n die oben angegebenen Bedeutungen haben, bei erhöhter Temperatur unter Schutzgas in Gegenwart eines Katalysators umsetzt.

Die Erfindung betrifft außerdem die Verwendung der Hydroxyalkylpolyethylen- und Hydroxyalkylpolypropylenglykolether der allgemeinen Formel (I) oder ihrer Mischungen als schaumdrückende Zusätze in schaumarmen Reinigungsmitteln.

Die erfindungsgemäßen Hydroxyalkylpolyethylen- und Hydroxyalkylpolypropylenglykolether entsprechen der allgemeinen Formel (I)

$$\overset{OH}{\overset{|}{R^1-CH}}-CH_2-(\overset{R^3}{\overset{|}{OCH}}-CH_2)_n-OR^2 \qquad (I)$$

In der allgemeinen Formel (I) steht $R^1$ für einen linearen Alkylrest mit 6 bis 16 C-Atomen. Es kommen also für die erfindungsgemäßen Verbindungen als Substituent $R^1$ die Reste n-Hexyl, n-Heptyl, n-Octyl, n-Nonyl, n-Decyl, n-Undecyl, n-Dodecyl, n-Tridecyl, n-Tetradecyl, n-Pentadecyl und n-Hexadecyl in Frage. Bevorzugt steht der Substituent $R^1$ in der oben genannten allgemeinen Formel (I) für lineare Alkylreste mit C-Atom-Zahlen im Bereich von 8 bis 12.

In der oben genannten allgemeinen Formel (I) steht $R^2$ erfindungsgemäß für einen linearen oder verzweigten, gesättigten oder ungesättigten Alkylrest mit 1 bis 22 C-Atomen. Es kommen somit als Substituent $R^2$ die Reste Methyl, Ethyl, n-Propyl, n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octydecyl, n-Nonadecyl, n-Eicosyl, n-Uneicosyl und n-Docosyl sowie die verzweigtkettigen Isomeren der genannten Alkylreste in Frage. $R^2$ kann auch anstelle der gesättigten Alkylreste ungesättigte Alkylreste mit einer Zahl von C-Atomen im oben genannten Bereich bedeuten. Auch derartige ungesättigte Alkylreste (Alkenylreste) können erfindungsgemäß linear oder verzweigt sein.

In einer bevorzugten Ausführungsform steht $R^2$ für lineare, gesättigte oder ungesättigte Alkylreste mit 1 bis 22 C-Atomen, von denen die linearen gesättigten Alkylreste mit 1 bis 22 C-Atomen weiter bevorzugt sind.

In einer besonders bevorzugten Ausführungsform werden Hydroxyalkylpolyethylen- und Hydroxyalkylpolypropylenglykolether der allgemeinen Formel (I) beansprucht, in der $R^2$ für lineare, gesättigte Alkylreste mit 1 bis 12 C-Atomen, bevorzugt für solche Alkylreste mit 3 bis 6 C-Atomen, steht.

In der oben genannten allgemeinen Formel (I), die die erfindungsgemäßen Verbindungen repräsentiert, steht $R^3$ für Wasserstoff oder eine Methylgruppe, wobei die Bedeutung "Wasserstoff" für $R^3$ bevorzugt ist.

In der allgemeinen Formel (I) hat n die Bedeutung einer Zahl von 0 bis 30, wobei der Bereich von 1 bis 10 für n bevorzugt ist. Dies bedeutet, daß in den erfindungsgemäßen Hydroxyalkylpolyethylen- und Hydroxyalkylpolypropylenglykolethern 0 bis 30 Ethoxy- oder Propoxyreste, bevorzugt 1 bis 10 Ethoxy- bzw. Propoxyreste in die Molekülkette eingebaut sind. Es ist jedoch auch möglich, daß in den genannten Verbindungen Ethoxyreste und Propoxyreste nebeneinander in beliebigem Verhältnis und beliebiger Reihenfolge in die Molekülkette eingebaut sind; derartige gemischte Ether fallen ebenfalls unter die allgemeine Formel (I).

Die erfindungsgemäßen Verbindungen der allgemeinen Formel (I) sind - was mit der vorliegenden Erfindung angestrebt war - gut biologisch abbaubar und erfüllen damit die geltenden gesetzlichen Vorschriften; eine Beeinträchtigung der Abwässer bei Verwendung der erfindungsgemäßen Verbindungen der allgemeinen Formel (I) ist somit nicht zu befürchten.

Der wesentlichste Vorteil der erfindungsgemäßen Verbindungen ist jedoch, daß sie hinsichtlich der ent schäumenden Wirkung erhebliche Verbesserungen gegenüber den bisher bekannten Verbindungen des Standes der Technik aufweisen. So wird bei Einsatz der erfindungsgemäßen Hydroxy-Mischether weniger Anfangsschaum als mit schon aus dem Stand der Technik bekannten Verbindungen gebildet. Andererseits ist das Durchhaltevermögen der Verbindungen deutlich besser.

Die Hydroxyalkylpolyethylen- und Hydroxyalkylpolypropylenglykolether der allgemeinen Formel (I) sowie auch ihre Mischungen werden nach einem optimierten Verfahren hergestellt. Dieses ist dadurch gekennzeichnet, daß man Epoxide der allgemeinen Formel (II)

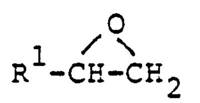

(II)

in der $R^1$ die oben angegebenen Bedeutungen hat, mit Alkoholalkoxylaten der allgemeinen Formel (III)

$$H-(O\overset{R^3}{\overset{|}{C}}H-CH_2)_n-OR^2 \qquad (III)$$

in der $R^2$ und $R^3$ sowie n die oben angegebenen Bedeutungen haben, bei erhöhter Temperatur unter Schutzgas in Gegenwart eines Katalysators umsetzt.

Ausgangsstoffe des Verfahrens zur Herstellung der Verbindungen der allgemeinen Formel (I) sind also einerseits Epoxide der allgemeinen Formel (II). Derartige Epoxide entstehen aus auf petrochemischem Wege in großen Mengen erhältlichen α-Olefinen, in der Mehrzahl der Fälle Monoolefinen, durch an sich bekannte Epoxidationsreaktionen, beispielsweise durch Umsetzungen der genannten Olefine mit Percarbonsäuren oder ähnlichen, Epoxide bildenden Reagenzien.

Reaktionspartner der oben beschriebenen Epoxide der allgemeinen Formel (II) sind Alkoholalkoxylate der allgemeinen Formel (III). Derartige Verbindungen (III) entstehen auf an sich bekanntem Wege aus Alkoholen und Olefinepoxiden. Im vorliegenden Fall werden unter Alkoholalkoxylaten der allgemeinen Formel (III) die Verbindungen verstanden, die aus den entsprechenden Alkoholen und Ethylenoxid bzw. Propylenoxid entstehen. Bevorzugte Reaktionspartner in dem vorliegenden Verfahren sind Alkoholalkoxylate der allgemeinen Formel (III), in der $R^2$ für lineare, gesättigte oder ungesättigte Alkylreste mit 1 bis 22 C-Atomen steht. Die für diese Bedeutung von $R^2$ möglichen Alkylreste sind oben im Zusammenhang mit den Verbindungen der allgemeinen Formel (I) aufgeführt. Von diesen Verbindungen sind diejenigen mit Vorteil einsetzbar, in deren allgemeiner Formel (III) $R^2$ für einen linearen gesättigten Alkylrest mit 1 bis 22 C-Atomen steht. Mit besonderem Vorteil sind die Verbindungen verwendbar, in denen $R^2$ in der allgemeinen Formel (III) einen gesättigten linearen Alkylrest mit 1 bis 12 C-Atomen, bevorzugt mit 3 bis 6 C-Atomen bedeutet. Das heißt im Rahmen der vorliegenden Erfindung, daß als Edukte für die Herstellung der erfindungsgemäß einzusetzenden Alkoholalkoxylate der allgemeinen Formel (III) am meisten bevorzugt Alkohole mit 3 bis 6 C-Atomen mit Alkylenoxiden wie Ethylenoxid oder Propylenoxid umgesetzt werden, wobei dann die besonders bevorzugten Alkoholalkoxylate der allgemeinen Formel (III) entstehen. Darunter

fallen also in diesem Fall Alkohole aus der Gruppe n-Propanol, n-Butanol, n-Pentanol und n-Hexanol. Bevorzugt sind in diesem Fall die Umsetzungsprodukte mit Ethylenoxid, so daß in den erfindungsgemäß einsetzbaren Alkoholalkoxylaten (III) $R^3$ bevorzugt die Bedeutung "H" hat. Das Umsetzungsverhältnis, das auch letztlich die Zahl der Alkoxygruppen im Molekül der Verbindungen der allgemeinen Formel (III) bestimmt, liegt im Bereich 1 : 1 bis 1 : 15, so daß - sofern n in der oben genannten allgemeinen Formel (III) nicht gleich Null ist - n einen Wert im Bereich von 1 bis 15 annimmt. Ein bevorzugter Bereich ist jedoch der von 1 bis 10.

Zum Einsatz in dem erfindungsgemäßen Verfahren sind auch solche Alkoholalkoxylate geeignet, in denen die wiederkehrende Einheit aus Ethoxy- und Propoxyresten in beliebigem Verhältnis und in beliebiger Reihenfolge in der Kette besteht, solange die Zahl n für die wiederkehrenden Einheiten im oben genannten Bereich liegt.

Entsprechend dem erfindungsgemäßen Verfahren zur Herstellung der Verbindungen der allgemeinen Formel (I) wird bei der Umsetzung von (II) mit (III) ein Molverhältnis im Bereich von ca. 1 : 1 eingestellt. Die Umsetzung wird bei erhöhter Temperatur durchgeführt. Dies bedeutet erfindungsgemäß, daß das Reaktionsgemisch unter Schutzgas auf Reaktionstemperaturen im Bereich von 100 bis 180 °C erhitzt wird, bei denen dann die Umsetzung in hinreichender Geschwindigkeit mit befriedigender Ausbeute stattfindet. Dabei wird ein Temperaturbereich von 120 bis 160 °C bevorzugt.

Erfindungsgemäß wird das Verfahren zur Herstellung der Hydroxyalkylpolyethylen- und Hydroxyalkylpolypropylenglykole der allgemeinen Formel (I) in Gegenwart eines Katalysators durchgeführt. Ausreichend zur Durchführung der Reaktion ist eine Katalysatormenge im Bereich von 0,01 bis 2,0 Gew.-%, bezogen auf das Gesamtgewicht des Reaktionsgemisches. Es können erfindungsgemäß sowohl saure als auch basische Katalysatoren verwendet werden. Bevorzugte Katalysatorsysteme sind Alkalimetallalkoholate, Mineralsäuren wie $H_2SO_4$ oder Lewis-Säuren wie $BF_3$-Etherat, wobei in dem erfindungsgemäßen Verfahren Natriummethanolat besonders bevorzugt wird.

Das Verfahren gemäß der Erfindung wird durch das nachfolgende Reaktionsschema verdeutlicht.

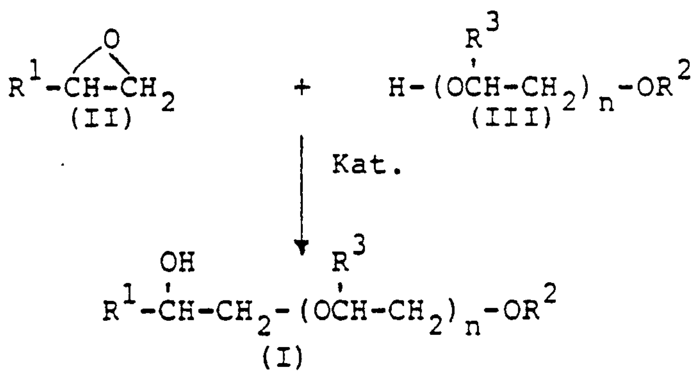


Wie voranstehend schon angedeutet wurde, entstehen bei dem erfindungsgemäßen Verfahren entsprechend dem vorstehenden Reaktionsschema nicht nur Einzelverbindungen der allgemeinen Formel (I), sondern auch Mischungen derartiger Verbindungen. So führt die Umsetzung der Alkohole mit Alkylenoxiden in bestimmten Molverhältnissen nicht zu einem einheitlichen Produkt (III); vielmehr entstehen Produktgemische verschiedener Verbindungen (III) mit einer mehr oder weniger breiten Verteilung der Zahl der Alkoxygruppen im Molekül, d.h. einer mehr oder weniger breiten Streuung von n in der allgemeinen Formel (III). Der Einsatz von Gemischen der Alkoholalkoxylate (III) in das erfindungsgemäße Verfahren muß dann auch ein Gemisch von Produkten (I) zur Folge haben. Auch solche Produktgemische sowie Verfahren zur Herstellung derartiger Gemische werden von der vorliegenden Erfindung mit umfaßt.

Gegenüber Verfahren aus dem Stand der Technik weist das vorliegende Verfahren - abgesehen von der Bildung neuer und gegenüber dem Stand der Technik verbesserter Produkte (I) - den wesentlichen Vorteil auf, daß bei der beschriebenen, erfindungsgemäßen Vorgehensweise zur Herstellung der Hydroxy-Mischether die Reaktionszeiten des Standes der Technik bei gleichbleibender Qualität und Quantität der Ausbeute von etwa 6 auf 0,5 bis 1,5 h reduziert werden können. Dies bedeutet eine wesentliche Beschleunigung der Verfahrensführung bei quantitativen Ausbeuten.

Die wie oben beschrieben herstellbaren Verbindungen der allgemeinen Formel (I) oder auch die verfahrensgemäß erhaltenen Mischungen derartiger Verbindungen werden als schaumdrückende Zusätze in schaumarmen Reinigungsmitteln für industrielle und gewerbliche Zwecke angewendet. Sie haben in derartigen Reinigungsmitteln den großen Vorteil, eine deutlich unter den bisherigen Standards liegende

Menge an Anfangsschaum mit einem hohen Durchhaltevermögen zu verbinden. Die Verwendung der Verbindungen (I) ist auch deswegen vorteilhaft, weil die Reinigungsmittel, die derartige schaumdrückende Zusätze enthalten, die geltenden gesetzlichen Vorschriften hinsichtlich der biologischen Abbaubarkeit erfüllen.

Die Erfindung wird durch die nachfolgenden Beispiele näher erläutert.

Beispiel 1

354,6 g (3,0 Mol) Butylglykol und 6,5 g Natriummethanolat (30 %ige Lösung in Methanol) wurden zwecks Entfernung des mit dem Katalysator eingebrachten Methanols im Vakuum auf 60 °C erwärmt. Nach Zugabe von 390,3 g (3,0 Mol) 1,2-Epoxyoctan (Epoxidzahl = 6,4) wurde 130 min auf 160 °C erhitzt. Die Epoxidzahl der Endprobe betrug 0,15, die OHZ betrug 248,6.

Beispiel 2

283,9 g (1,75 Mol) Butyldiglykol und 7,5 g Natriummethanolat (30 %ige Lösung in Methanol) wurden im Vakuum bei 60 °C erwärmt, um Methanol zu entfernen. Man gab 449,4 g (1,75 Mol) 1,2-Epoxyhexadecan zu und erhitzte unter Rühren 60 min auf 160 °C. Die Epoxidzahl der Endprobe betrug 0,23, die OHZ 143,2.

Beispiel 3

355,3 g (1,25 Mol) eines Anlagerungsproduktes von 5 Mol Ethylenoxid an 1 Mol Butanol (Butanol-5EO) und 3,3 g Natriummethanolat (30 %ige Lösung in Methanol) wurden im Vakuum bei 60 °C erwärmt, um Methanol zu entfernen. Nach Zugabe von 197,0 g (1,25 Mol) 1,2-Epoxydecan wurde 15 min auf 160 °C erhitzt. Die Epoxidzahl der Endprobe betrug 0,11, die OHZ 134,5.

Beispiel 4

241,0 g (0,5 Mol) Butanol-10EO und 1 g Natriummethanolat (30 %ige Lösung in Methanol) wurden vorge legt und 10 min bei 70 °C im Wasserstrahlvakuum evakuiert. Unter Inertgasatmosphäre wurden 117,6 g (0,5 Mol) 1,2-Epoxytetradecan zugegeben und 420 min auf 110 °C erhitzt und anschließend mit wenigen Tropfen konzentrierter Schwefelsäure neutralisiert. Man erhielt 348 g einer goldgelben Flüssigkeit. Die Epoxidzahl der Probe betrug 0,4, die OHZ 95,8 und der Trübungspunkt in Wasser 16,7 °C

Beispiel 5

277,2 g (0,58 Mol) Butanol-10EO, 135,2 g (0,58 Mol) 1,2-Epoxytetradecan und 2,9 g konzentrierte Schwefelsäure wurden zusammengegeben und unter Stickstoff 370 min auf 120 °C erhitzt. Anschließend wurde mit Natriummethanolat neutralisiert. Die Epoxidzahl des Produktes betrug 0,16, die OHZ 95,9.

Beispiel 6

241,0 g (0,5 Mol) Butanol-10EO und 117,6 g (0,5 Mol) 1,2-Epoxytetradecan wurden vorgelegt und bei 70 °C 10 min evakuiert. Das Vakuum wurde mit Argon aufgehoben und 4,3 ml (= 4,88 g) einer 48 %igen Bortrifluoridlösung in Ether zugegeben. Es wurde 15 min auf 140 °C erhitzt und anschließend mit Natriummethanolatlösung neutralisiert und filtriert. Die OHZ der Endprobe betrug 81,9 (Theorie: 78,2).

Beispiel 7

In ähnlicher wie der in den Beispielen 1 bis 6 beschriebenen Weise wurden weitere Hydroxyalkylpolyethylen- bzw. Hydroxyalkylpolypropylenglykolether (I) hergestellt, die der nachfolgenden Tabelle 1 zu entnehmen sind.

Tabelle 1

Weitere Verbindungen der Formel

$$R^1-\overset{OH}{\overset{|}{C}H}-CH_2-(O\overset{R^3}{\overset{|}{C}H}-CH_2)_n-OR^2 \qquad (I)$$

$(R^3 = H$[1]$)$

| Bsp. 7 | $R^1$ | n | $R^2$ | OHZ | Epoxidzahl |
|---|---|---|---|---|---|
| a | $C_{14}H_{29}$ | 5 | (Oleyl) | 85 | < 0,2 |
| b | $C_{10}H_{21}$ | 9[1] | $C_{12/14}H_{25/29}$ | 90 | < 0,2 |
| c | $C_{12}H_{25}$ | 7 | $C_{12/13}H_{25/27}$ | 87 | < 0,2 |
| d | $C_{14}H_{29}$ | 7 | $C_{12/13}H_{25/27}$ | 82 | < 0,2 |
| e | $C_{10}H_{21}$ | 2 | $C_4H_9$ | 184 | < 0,2 |
| f | $C_{12}H_{25}$ | 2 | $C_4H_9$ | 167 | < 0,2 |
| g | $C_{14}H_{29}$ | 2 | $C_4H_9$ | 160 | < 0,2 |
| h | $C_{16}H_{33}$ | 2 | $C_4H_9$ | 160 | < 0,2 |
| i | $C_{16}H_{33}$ | 5 | $C_4H_9$ | 107 | < 0,2 |
| j | $C_{12}H_{25}$ | 10 | $C_4H_9$ | 86 | < 0,2 |
| k | $C_{14}H_{29}$ | 10 | $C_4H_9$ | 81 | < 0,2 |
| l | $C_{16}H_{33}$ | 10 | $C_4H_9$ | 76 | < 0,2 |

Erläuterungen: [1] in Verbindung des Bsp. 7b:
$R^3$ = H bzw. $CH_3$;
Molverh. EO : PO = 4 : 5

Beispiel 8 und Vergleichsbeispiele 1 und 2

Prüfung der Antischaumwirkung

Die Antischaumwirkung der erfindungsgemäßen Hydroxyalkylpolyethylen- und Hydroxyalkylpolypropylenglykolether der allgemeinen Formel (I) wurde mit einer nach DIN 53 902 genormten Schaumschlagapparatur nach Götte gemessen. Dazu wurden bei 65 °C jeweils 200 ml einer Testlösung, die 1 Gew.-% Natriumhydroxid und 0.03 Gew.-% (300 ppm) des jeweiligen Entschäumers enthielt, in der Schaumschlagapparatur geprüft. Den Lösungen wurde als Testschäumer eine 25 Gew.-%ige Lösung des Diethanolaminsalzes des Tetrapropylenbenzolsulfonats in steigenden Mengen zugesetzt.

Als Vergleichsprodukte (Standards) diente ein kommerziell unter dem Namen "Genapol PN 30" (Hoechst AG) erhältliches, biologisch nur ungenügend abbaubares Addukt von 30 EO und 60 PO an Ethylendiamin (Vergleichsbeispiel 1) sowie ein ebenfalls kommerziell unter dem Namen "Dehypon LT 104" (Henkel KGaA) erhältliches, biologisch abbaubares, endgruppenverschlossenes Addukt von Ethylenoxid an einen Fettalkohol (Vergleichsbeispiel 2).

Für die Beurteilung der Antischaumwirkung wurden als Kriterien herangezogen:

1. Der Anfangsschaum: dieser muß möglichst gering sein. Bei einem Zusatz von 1000 ppm Testschäumer sollte unter den standardisierten Prüfbedingungen ein Anfangsschaumvolumen von 40 ml nicht überschritten werden.

2. Das sogenannte "Durchhaltevermögen": dieses sollte möglichst groß sein. Darunter wird diejenige Testschäumerkonzentration in ppm verstanden, bei der das Schaumvolumen unter den standardisierten Bedingungen und einer gegebenen Konzentration an Entschäumer 300 ml beträgt oder überschreitet. Ein Durchhaltevermögen von mindestens 1800 ppm (dies entsprach dem mit den Vergleichsprodukten erhaltenen Wert) sollte gegeben sein.

Zur Prüfung der Antischaumwirkung wurde das Schaumvolumen (in ml) jeweils 5 sec nach einer Serie von 500 Schlägen abgelesen. Es wurde ein Durchschnittswert aus fünf Einzelmessungen ermittelt.

Die Ergebnisse sind der nachfolgenden Tabelle 2 zu entnehmen.

Tabelle 2

| Bsp. | Anfangsschaum (ml) | Durchhaltevermögen (ppm Testschäumer) |
|---|---|---|
| 7a | 20 | 2 400 |
| 7b | 20 | 2 200 |
| 7c | 30 | 2 000 |
| 7d | 30 | 2 400 |
| 7e | 30 | 2 600 |
| 7f | 30 | 4 200 |
| 7g | 20 | 3 200 |
| 7h | 40 | 3 600 |
| 7i | 15 | 1 800 |
| 7j | 30 | 2 000 |
| 7k | 30 | 1 800 |
| 7l | 35 | 1 800 |
| Vgl. 1 | 30 | 1 800 |
| Vgl. 2 | 40 | 1 800 |

Erbebnis:

Wie Tabelle 2 zu entnehmen ist, zeigen die erfindungsgemäßen Hydroxy-Mischether der allgemeinen Formel (I) in bezug auf Anfangsschaumvolumen und Durchhaltevermögen deutlich bessere Ergebnisse als bislang beschriebene Entschäumer.

**Ansprüche**

1. Hydroxyalkylpolyethylen- und Hydroxyalkylpolypropylenglykolether der allgemeinen Formel (I)

$$R^1-\underset{}{\overset{OH}{CH}}-CH_2-(O\overset{R^3}{CH}-CH_2)_n-OR^2 \qquad (I)$$

in der
R' für einen linearen Alkylrest mit 6 bis 16 C-Atomen,
$R^2$ für einen geradkettigen oder verzweigten, gesättigten oder ungesättigten Alkylrest mit 1 bis 22 C-Atomen,
$R^3$ für Wasserstoff oder eine Methylgruppe und n für eine Zahl von 0 bis 30
stehen, sowie Mischungen mehrerer derartiger Verbindungen.

2. Verbindungen der allgemeinen Formel (I) nach Anspruch 1, in der $R^1$ für einen linearen Alkylrest mit 8 bis 12 C-Atomen steht.

3. Verbindungen der allgemeinen Formel (I) nach Ansprüchen 1 und 2, in der $R^2$ für lineare, gesättigte oder ungesättigte Alkylreste, bevorzugt für lineare gesättigte Alkylreste, mit 1 bis 22 C-Atomen steht.

4. Verbindungen der allgemeinen Formel (I) nach Anspruch 3, in der $R^2$ für lineare gesättigte Alkylreste mit 1 bis 12 C-Atomen, bevorzugt mit 3 bis 6 C-Atomen, steht.

5. Verbindungen der allgemeinen Formel (I) nach Ansprüchen 1 bis 4, in der $R^3$ für Wasserstoff steht.

6. Verbindungen der allgemeinen Formel (I) nach Ansprüchen 1 bis 5, in der n für eine Zahl im Bereich von 1 bis 10 steht.

7. Verfahren zur Herstellung von Hydroxyalkylpolyethylen- und Hydroxyalkylpolypropylenglykolethern der allgemeinen Formel (I)

$$R^1-\overset{OH}{CH}-CH_2-(O\overset{R^3}{CH}-CH_2)_n-OR^2 \qquad (I)$$

in der
R' für einen linearen Alkylrest mit 6 bis 16 C-Atomen,
$R^2$ für einen geradkettigen oder verzweigten, gesättigten oder ungesättigten Alkylrest mit 1 bis 22 C-Atomen,
$R^3$ für Wasserstoff oder eine Methylgruppe und
n für eine Zahl von 0 bis 30
stehen, sowie von Mischungen mehrerer derartiger Verbindungen, dadurch gekennzeichnet, daß man Epoxide der allgemeinen Formel (II)

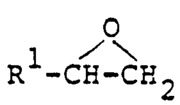

(II)

in der $R^1$ die oben angegebenen Bedeutungen hat, mit Alkoholalkoxylaten der allgemeinen Formel (III)

$$H-(O\overset{R^3}{CH}-CH_2)_n-OR^2 \qquad (III)$$

in der $R^2$ und $R^3$ sowie n die oben angegebenen Bedeutungen haben, bei erhöhter Temperatur unter Schutzgas in Gegenwart eines Katalysators umsetzt.

8. Verfahren nach Anspruch 7, dadurch gekennzeichnet, daß man Epoxide der allgemeinen Formel (II), in der R' für einen linearen Alkylrest mit 8 bis 12 C-Atomen steht, in die Reaktion einsetzt.

9. Verfahren nach Anspruch 7, dadurch gekennzeichnet, daß man Alkoholalkoxylate der allgemeinen Formel (III) in die Reaktion einsetzt, in der $R^2$ für lineare, gesättigte oder ungesättigte Alkylreste, bevorzugt für lineare gesättigte Alkylreste, mit 1 bis 22 C-Atomen steht.

10. Verfahren nach Anspruch 9, dadurch gekennzeichnet, daß man Verbindungen der allgemeinen Formel (III) in die Reaktion einsetzt, in der $R^2$ für lineare gesättigte Alkylreste mit 1 bis 12 C-Atomen, bevorzugt mit 3 bis 6 C-Atomen, steht.

11. Verfahren nach Ansprüchen 9 und 10, dadurch gekennzeichnet, daß man Verbindungen der allgemeinen Formel (III) in die Reaktion einsetzt, in der $R^3$ für H steht.

12. Verfahren nach Ansprüchen 9 bis 11, dadurch gekennzeichnet, daß man Verbindungen der allgemeinen Formel (III) in die Reaktion einsetzt, in der n für eine Zahl im Bereich von 1 bis 10 steht.

13. Verfahren nach Ansprüchen 7 bis 12, dadurch gekennzeichnet, daß man für die Umsetzung der Verbindungen der allgemeinen Formel (II) mit den Verbindungen der allgemeinen Formel (III) ein Molverhältnis (II) : (III) im Bereich von ca. 1 : 1 wählt.

14. Verfahren nach Ansprüchen 7 bis 13, dadurch gekennzeichnet, daß man die Reaktion bei Temperaturen im Bereich von 100 bis 180 °C, bevorzugt im Bereich von 120 bis 160 °C durchführt.

15. Verfahren nach Ansprüchen 7 bis 14, dadurch gekennzeichnet, daß man einen Katalysator in einer Menge von 0,01 bis 2,0 Gew.-%, bezogen auf das Gesamtgewicht des Reaktionsgemisches, verwendet.

16. Verfahren nach Anspruch 15, dadurch gekennzeichnet, daß man als Katalysator Alkalimetallalkoholate, Schwefelsäure oder Bortrifluoridetherat, bevorzugt Natriummethanolat, verwendet.

17. Verwendung der Hydroxyalkylpolyethylen- und Hydroxyalkylpolypropylenglykolether der allgemeinen Formel (I) nach Ansprüchen 1 bis 6 oder ihrer Mischungen als schaumdrückende Zusätze in schaumarmen Reinigungsmitteln.